# EUROPÄISCHE PATENTSCHRIFT

(11) **EP 0 325 771 B1**
(45) Veröffentlichungstag und Bekanntmachung des Hinweises auf die Patenterteilung: **08.09.1993**
(21) Anmeldenummer: 88121357.3
(22) Anmeldetag: 21.12.1988
(51) Int. Cl.: A61F 7/00, A61F 5/00, A61H 7/00, A61H 33/00

(54) **Vorrichtung zur Behandlung von Körperteilen des Menschen**
Treatment device for parts of the human body
Dispositif pour le traitement des parties du corp humain

(30) Priorität: 25.01.1988 DE 3802010
(43) Veröffentlichungstag der Anmeldung: 02.08.1989
(73) Patentinhaber: Siegel, Gunter, 72160 Horb (DE)
(72) Erfinder: Siegel, Gunter, 72160 Horb (DE)
(74) Vertreter: Dauster, Hanjörg, Dipl.-Ing.

(56) Entgegenhaltungen:
- EP-A- 0 061 843
- WO-A-84/01904
- DE-A- 2 352 092
- DE-C- 558 180

## Beschreibung

Die Erfindung betrifft eine Vorrichtung zum Behandeln von Körperteilen eines Menschen nach dem Oberbegriff des Patentanspruches 1.

Derartige Vorrichtungen werden eingesetzt, um an erkrankten Körperteilen eine Heilwirkung zu erzeugen. Dies wird durch ein Behandlungsmedium erreicht, das gezielt mit dem krankhaften Körperteil in Kontakt gebracht werden kann. Im Bereich des krankhaften Körperteiles kann hierdurch die Durchblutung gezielt erhöht oder vermindert werden. Vorrichtungen dieser Art kommen bei verschiedenen Therapiemethoden zum Einsatz. Hier ist insbesondere die Kneippsche-, Hypo-, Hyper- oder Kryo-Therapie zu nennen. Bei Gelenk- und Muskelschmerzen kann mit einer derartigen Vorrichtung eine Schmerzlinderung und eine Beschleunigung des Heilprozesses bewirkt werden. Weiterhin kommen derartige Vorrichtungen bei der Wundspülung, Abzessbehandlung und zur Therapie von verschiedenartigen Hautkrankheiten zum Einsatz.

Aus der DE-C-15 66 382 ist eine Vorrichtung bekannt, die eine Einrichtung besitzt, die auf dem zu behandelnden Körperteil aufgebracht wird und an der eine Vielzahl von über die Fläche ihrer Innenwandung verteilten Öffnungen angeordnet sind. An der Einrichtung sind Hohlelemente vorgesehen,die an der der zu behandelnden Körperfläche zugewandten Seite Öffnungen und Vorsprünge besitzen und die der zu behandelnden Körperoberfläche angepaßt sind. Ein Behandlungsmedium, das wahlweise temperiert werden kann, wird dem Körperteil über die an den Hohlelementen vorgesehenen Öffnungen zugeführt, in einen Sammelbehälter aufgefangen und mittels einer Pumpe im Kreislauf geführt.

Aus der FR-A-776 908 ist eine Vorrichtung bekannt, die eine Saugeinrichtung besitzt, die auf einem zu behandelnden Körperteil aufsetzbar ist. Die Saugeinrichtung baut sich aus einem Außen- und einem Innenbehälter auf. Eine Zulaufleitung für das Behandlungsmedium ist an dem Innenbehälter und eine Rücklaufleitung an dem Außenbehälter angeordnet. Der Anschluß einer Saugpumpe an die Rücklaufleitung bewirkt einerseits das Festsaugen der Saugeinrichtung auf dem zu behandelnden Körperteil und andererseits ein Ansaugen des Behandlungsmediums über die Zulaufleitung. Das Behandlungsmedium wird an das Körperteil geführt und kann über einen zwischen dem Außen- und Innenbehälter liegenden Ringraum entweichen.

Eine andere Saugeinrichtung (DE-C-383 270) soll für die Wundbehandlung eingesetzt werden. Hierzu ist ein die Wunde umschließender Hohlkörper vorgesehen, der Öffnungen für den Zufluß und Abfluß einer Spülflüssigkeit enthält. Der Hohlkörper ist mit einer Saugvorrichtung verbunden, die ein fortwährendes Auspumpen des Hohlkörpers und ein ununterbrochenes Einziehen der Spülflüssigkeit bewirkt. Durch die Saugwirkung der abfließenden Flüssigkeit wird gleichzeitig das Festsaugen des Apparates am Körperteil bewirkt.

Bei einer weiteren bekannten Vorrichtung zur Hitze- und Massagebehandlung (DE-C-558 180) ist eine Wasserstrahlpumpe vorgesehen, durch die Wasserstrahlen mit erhöhter Temperatur unvermischt oder vermischt mit Luft und anderen Gasen durch eine oder mehrere Düsen unmittelbar auf die zu behandelnde Körperstelle gespritzt werden kann. Eine Ausführung der bekannten Bauart beschreibt eine an den zu behandelnden Körperteil anordenbare Saugeinrichtung, die sich durch den mittels einer Pumpe bewirkten Unterdruck selbsttätig festsaugt. Das Behandlungsmedium wird im Kreislauf geführt und kann mittels einer Heizeinrichtung erwärmt werden. Die Vorrichtung gemäß der DE-C-558 180 liegt dem Oberbegriff des Anspruchs zugrunde.

Der Erfindung liegt die Aufgabe zugrunde, eine Vorrichtung der eingangs genannten Art so auszubilden, daß ein weiteres Behandlungsmedium, das eine andere Temperatur besitzt, dem zu behandelnden Körperteil zugeführt werden kann, wobei keine Vermischung der beiden Behandlungsmedien auftritt.

Diese Aufgabe wird bei einer gattungsgemäßen Vorrichtung durch die kennzeichnenden Merkmale des Patentanspruches 1 gelöst. Mit der neuen Vorrichtung können einem zu behandelnden Körperteil unterschiedlich temperierte Behandlungsmedien zugeführt werden, indem das eine Behandlungsmedium erwärmt und das andere Behandlungsmedium gekühlt ist. Die taktweise Zuführung auf das zu behandelnde Körperteil bewirkt einen besonders intensiven Therapieeffekt. Insbesondere kann die Durchblutung mit einer derartigen Vorrichtung im Bereich der zu behandelnden Körperstelle wesentlich gesteigert oder vermindert werden. Ein wesentlicher Vorteil der neuen Vorrichtung besteht darin, daß die beiden Behandlungsmedien in voneinander getrennten Kreisläufen geführt werden. Durch ein an der Saugeinrichtung angeordnetes Belüftungsventil kann Luft in die Saugeinrichtung eingesaugt werden, so daß eine vollständige Absaugung gelingt und keine Vermischung der beiden Behandlungsmedien auftritt. Die Behandlungsmedien können im Kreislauf geführt werden, wodurch der Energiebedarf der neuen Vorrichtung besonders gering ist. Hierdurch können die notwendigen Heiz- und Kühlvorrichtungen kleiner dimensioniert werden.

Eine vorteilhafte Ausführungsform sieht vor, daß der Zulaufanschluß in einem am tiefsten liegenden Bereich der Saugeinrichtung und der Sauganschluß in einem am höchsten liegenden Bereich der Saugeinrichtung angeordnet sind. Hierdurch wird eine vollständige Füllung der Saugeinrichtung mit dem jeweiligen Behandlungsmedium erreicht. Dies führt zu einer gleichmäßigen Therapiewirkung an dem zu behandelnden Körperteil.

Es ist vorteilhaft, wenn das Belüftungsventil im Bereich des Sauganschlusses angeordnet ist. Beim Wechseln vom ersten Behandlungsmedium zum zweiten Behandlungsmedium wird über das Belüftungsventil Luft angesaugt. Erst wenn die Saugeinrichtung vollständig vom ersten Behandlungsmedium geleert ist, wird das zweite Behandlungsmedium angesaugt.

Eine vorteilhafte Weiterbildung sieht vor, daß das Belüftungsventil zum Öffnen und Schließen von zwei Belüftungsbohrungen mit verschiedenem Querschnitt ausgebildet ist. Über die kleinere Belüftungsbohrung kann zwischen den beiden Behandlungsmedien Luft derart angesaugt werden, daß die Saugwirkung der Saugeinrichtung an dem zu behandelnden Körperteil nicht wesentlich beeinträchtigt wird. Die größere Belüftungsbohrung dient beim Behandlungsende zur vollständigen Belüftung der Saugeinrichtung.

Eine vorteilhafte Ausgestaltung sieht vor, daß das Belüftungsventil eine zentral angeordnete Ventilnadel besitzt, die an einer in einem Schließstempel vorgesehenen kleinen Bohrung lösbar anliegt und im wesentlichen durch ihre Schwerkraft in Schließstellung gebracht ist. Wird die Ventilnadel aus der kleinen Bohrung gelöst, so fließt ein kleiner Luftstrom in die Saugeinrichtung ein. Der Schließstempel des Belüftungsventils liegt an einer großen Bohrung an, die an der Saugeinrichtung angebracht ist und in Schließstellung von einem Federelement gehalten ist, wobei die Betätigung des Schließstempels mittels der Ventilnadel und eines daran angeordneten Stiftes erfolgt. Somit kann mittels der Ventilnadel sowohl die kleinere Bohrung als auch die große Bohrung freigegeben werden. Hierdurch wird die Betätigung des Belüftungsventils erleichtert.

Eine weitere Ausgestaltung sieht vor, daß das Belüftungsventil ansteuerbar und/oder manuell betätigbar ausgebildet ist. Bei einem ansteuerbaren Belüftungsventil kann vorgesehen sein, daß im Belüftungsventil eine mit der Ventilnadel zusammenwirkende Induktionsspule angeordnet ist.

Zur Erleichterung der Bedienung kann eine Steuereinheit vorgesehen sein, mit der die Ventile, das Belüftungsventil und die Pumpe steuerbar sind. Hierdurch ist auch ohne geschultes Bedienungspersonal eine Eigentherapie des Patienten möglich.

Die an der neuen Vorrichtung vorgesehene Steuereinheit kann manuell betätigbar sein. Eine vorteilhafte Weiterbildung sieht vor, daß die Steuereinheit mit einer automatischen Ablaufsteuerung und/oder Programmsteuerung versehen ist. Hierdurch wird die Bedienung der neuen Vorrichtung besonders einfach. Außerdem ist die Einhaltung bestimmter Behandlungsabläufe gesichert.

Es kann vorteilhaft sein, wenn die Steuereinheit vom automatischen Betrieb auf manuellen Betrieb umschaltbar ist.

Eine vorteilhafte Ausführungsform sieht vor, daß die Steuereinheit eine Ablaufsteuerung besitzt,die in einer ersten Betriebsphase ein Absaugen der Luft aus der Saugeinrichtung und das Ansaugen des ersten Behandlungsmediums,in einer zweiten Betriebsphase ein Leersaugen der Saugeinrichtung bei teilweise geöffneten Belüftungsventil und in einer dritten Betriebsphase ein Ansaugen eines zweiten Behandlungsmediums bewirkt. Weiterhin kann vorgesehen sein, daß die Ablaufsteuerung am Behandlungsende das Belüftungsventil zunächst nur teilweise und dann zeitverzögert nach dem Absaugen des Behandlungsmediums vollständig öffnet.

Weitere Merkmale und Vorteile der Erfindung ergeben sich aus den in der Zeichnung dargestellten Ausführungsformen und deren nachfolgender Beschreibung und den Unteransprüchen. Es zeigen:
- Fig. 1: eine schematische Darstellung einer erfindungsgemäßen Ausführungsform mit einer Saugglocke,
- Fig. 2: eine vergrößerte Darstellung der Ausführungsform gemäß Fig. 1 im Bereich der Saugglocke,
- Fig. 3: ein Belüftungsventil bei einer anderen Ausführungsform der Erfindung,
- Fig. 4: eine schematische Darstellung einer weiteren Ausführungsform der Erfindung mit einer Saugröhre und
- Fig. 5: den Schnitt längs der Linie V-V in Fig. 4.

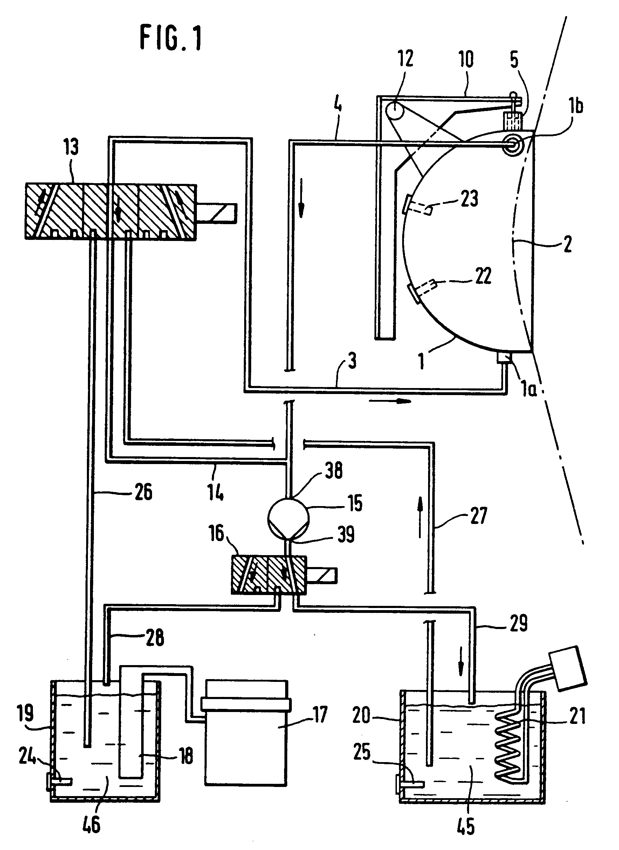

In Fig. 1 ist eine Saugglocke (1) dargestellt, die an einer Körperoberfläche (2) eines zu behandelnden Körperteiles angebracht ist. Die Saugglocke (1) besitzt in einem am tiefsten liegenden Bereich einen Zulaufanschluß (1a), der mit einer Vorlaufleitung (3) verbunden ist. In einem am höchsten liegenden Bereich der Saugglocke (1) ist ein Sauganschluß (1b) angeordnet, der mit einer Rücklaufleitung (4) verbunden ist.

Ein in einem Warmwasserbehälter (20) angeordnetes erstes Behandlungsmedium (45), das mittels eines in den Warmwasserbehälter (20) eintauchenden Wärmetauschers (21) erwärmt wird, kann mittels einer Pumpe (15) angesaugt werden. Die Pumpe (15) saugt zunächst die in der Saugglocke (1) vorhandene Luft ab, wodurch die Saugglocke (1) sich an die Körperoberfläche (2) ansaugt. Im Randbereich der Saugglocke (1) dichtet diese mit der Körperoberfläche (2) ab. Das erste Behandlungsmedium (45) wird über eine erste Steigleitung (27) angesaugt.Ein Mehrwegeventil (13), das elektromagnetisch ansteuerbar ist, nimmt hierbei eine erste Schaltstellung ein, bei der die Steigleitung (27) mit der Vorlaufleitung (3) verbunden ist. Nach dem Durchfließen des Mehrwegeventils (13) strömt das erste Behandlungsmedium (45) über die Vorlaufleitung (3) in die Saugglocke (1) ein. Das erste Behandlungsmedium (45) verläßt die Saugglocke (1) am Sauganschluß (1b) und wird über die Rücklaufleitung (4) zur Pumpe (15) geführt. Auf der Druckseite (39) der Pumpe (15) ist ein Zweiwegeventil (16) angeordnet, das ebenfalls als ansteuerbares elektromagnetisches Ventil ausgebildet ist. In einer ersten Schaltstellung des Zweiwegeventils (16) fließt das erste Behandlungsmedium (45) über die Ablaufleitung (29) wieder dem Warmwasserbehälter (20) zu. Somit wird das erste Behandlungsmedium (45) im Kreislauf geführt.

Ein zweites Behandlungsmedium (46) ist in einem Kaltwasserbehälter (19) eingefüllt. Das Behandlungsmedium (46) wird mittels eines Kältekompressors (17) gekühlt, dessen Verdampfer (18) im Innern des Kaltwasserbehälters (19) angeordnet ist.

Um eine Vermischung der beiden Behandlungsmedien (45, 46) zu vermeiden, wird zunächst das erste Behandlungsmedium (45) vollständig aus der Saugglocke (1) abgesaugt. Hierzu wird ein Belüftungsventil (5) betätigt, das im Bereich des Sauganschlusses (1b) an der Saugglocke (1) angeordnet ist. Das Belüftungsventil (5), auf dessen detaillierte Ausbildung weiter unten eingegangen wird, ermöglicht die gezielte Zuführung eines kleinen Luftstromes in die Saugglocke (1). Die über das Belüftungsventil (5) einströmende Luft verdrängt das erste Behandlungsmedium (45) vollständig. Hierbei bleibt die Saugwirkung der Saugglocke (1) an der Körperoberfläche (2) erhalten. Die vollständige Entleerung der Saugglocke (1) erfolgt durch Umstellen des Mehrwegeventils (13) in eine zweite Schaltstellung, bei der die Vorlaufleitung (3) mit einer Verbindungsleitung (14) verbunden wird. Hierdurch werden sowohl die Vorlaufleitung (3) als auch die Rücklaufleitung (4) zu Saugleitungen. Nach der Verdrängung des ersten Behandlungsmediums (45) aus der Saugglocke (1) werden die Ventile (13,16) angesteuert. In einer dritten Schaltstellung des Mehrwegeventils (13) besteht eine Verbindung zwischen der zweiten Steigleitung (26) und der Vorlaufleitung (3). Über diese gelangt das zweite Behandlungsmedium (46) in das Innere der Saugglocke (1). In gleicher Weise wie das erste Behandlungsmedium (45) wird auch das zweite Behandlungsmedium (46) über die Rücklaufleitung (4) von der Pumpe (15) abgesaugt. In der zweiten Schaltstellung des Zweiwegeventils (16) gelangt das zweite Behandlungsmedium (46) über die Ablaufleitung (28) in den Kaltwasserbehälter (19).

Am Ende der Behandlung wird das Mehrwegeventil (13) auf die zweite Schaltstellung gebracht, so daß eine Verbindung zwischen einer Verbindungsleitung (14) und der Vorlaufleitung (3) hergestellt ist. Die Verbindungsleitung (14) ist an der Saugseite (38) der Pumpe (15) mit der Rücklaufleitung (4) verbunden. Durch diese Schaltung des Mehrwegeventils (13) wird ermöglicht, daß das in der Saugglocke (1) enthaltene Medium gleichzeitig über die Vorlaufleitung (3) und die Rücklaufleitung (4) abgesaugt wird. Hierdurch wird eine besonders schnelle Absaugung möglich. Das Belüftungsventil (5) wird zunächst nur an seiner kleinen Bohrung (7) geöffnet, so daß in dieser Phase der Unterdruck an der Saugglocke (1) erhalten bleibt. Nachdem das Behandlungsmedium aus der Saugglocke (1) abgesaugt ist, kann das Belüftungsventil (5) vollständig geöffnet werden, wodurch der Unterdruck an der Saugglocke (1) auf Null sinkt und die Saugglocke (1) sich von der Körperoberfläche (2) löst. Danach wird die Pumpe (15) abgeschaltet.

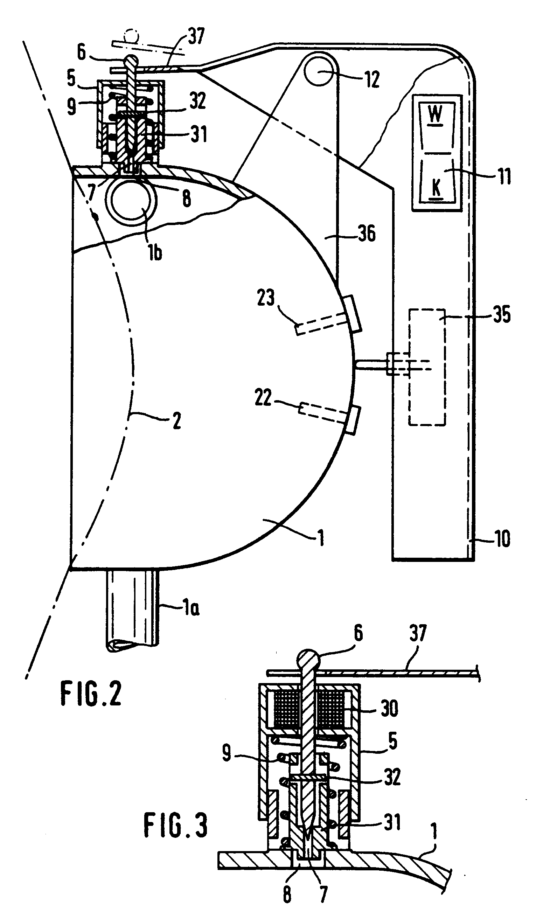

In Fig. 2 ist die Ausbildung der Saugglocke (1) detailliert dargestellt. Die Saugglocke (1) besitzt die Form einer Halbkugel. Die Öffnungsseite der Saugglocke (1) wird an dem zu behandelnden Körperteil angebracht.Die Positionierung der Saugglocke erfolgt derart, daß der Zulaufanschluß (1a) am tiefsten und der Sauganschluß (1b) am höchsten angeordnet ist. Im Bereich des Sauganschlusses (1b) ist das Belüftungsventil (5) angeordnet, das eine zentralgeführte Ventilnadel (6) besitzt. Die Ventilnadel (6) ist in einem Schließstempel (31) geführt, an dem eine kleine Belüftungsbohrung (7) ausgebildet ist. Bei einer axialen Verschiebung der Ventilnadel (6) wird die kleine Belüftungsbohrung (7) freigegeben. Hierdurch kann ein kleiner Luftstrom von der Außenseite in das Innere der Saugglocke (1) einströmen. Der Schließstempel (31) liegt an einer großen Belüftungsbohrung (8) an, die an der Saugglocke (1) ausgebildet ist. Die Betätigung des Schließstempels (31) erfolgt ebenfalls über die Ventilnadel (6). An dieser ist ein Stift (32) vorgesehen, der bei einer Axialverschiebung der Ventilnadel (6) mit dem Schließstempel (31) verbindbar ist. Die Öffnungsbewegung des Schließstempels (31) erfolgt gegen die Kraft eines Federelementes (9), das innerhalb des Belüftungsventiles (5) angeordnet ist.

Die Betätigung des Belüftungsventils (5) kann manuell über einen Hebel (10) erfolgen, der am oberen Ende des Ventilstempels (6) angreift. Bei einer kleinen Axialverschiebung der Ventilnadel (6) wird zunächst die kleine Bohrung (7) freigegeben. Bei weiterer Axialverschiebung gerät dann der Stift (32) der Ventilnadel (6) in Kontakt mit dem Schließstempel (31), so daß dieser von der großen Belüftungsbohrung (8) gelöst wird.

In Fig. 3 ist eine andere Ausführungsform eines Belüftungsventils (5) dargestellt. Dieses ist sowohl manuell als auch ansteuerbar betätigbar. Zur ansteuerbaren Betätigung ist innerhalb des Belüftungsventils (5) eine mit der Ventilnadel (6) zusammenwirkende Induktionsspule (30) vorgesehen. Hiermit kann die Ventilnadel (6) zunächst die Freigabe einer kleinen Belüftungsbohrung (7) und später einer großen Belüftungsbohrung (8) bewirken.

Der Hebel (10), der näherungsweise L-förmig ausgebildet ist, ist an einem Drehpunkt (12) eines Scharnierbocks (36) gelagert, der an der Saugglocke (1) befestigt ist. Eine Hebelverlängerung (37) des Hebels (10) ist mit dem Kopfende der Ventilnadel (6) verbunden. Eine Schwenkbewegung des Hebels (10) um das Drehlager (12) bringen die Ventilnadel (6) und das Kopfende (37) in die in Fig. 2 strichliniert eingezeichnete Lage. Am Hebel (10) ist weiterhin ein Schalter (11) angeordnet, mit dem die Ventile (13, 16) ansteuerbar ist. Somit wird eine manuelle Steuerung des Behandlungsablaufes ermöglicht. Weiterhin ist am Hebel (10) ein Pumpenschalter (35) angeordnet.

Bei der Vorrichtung gemäß Fig. 1 ist eine in der Zeichnung nicht dargestellte Steuereinheit vorgesehen, die von automatischen Betrieb auf manuellen Betrieb umschaltbar ist. Im manuellen Betrieb kann die Bedienperson den Behandlungsablauf frei wählen. Die Ansteuerung des Mehrwegeventils (13), des Zweiwegeventils (16), der Pumpe (15) und des Belüftungsventils (5) kann frei erfolgen. Die einzelnen Behandlungsschritte werden mit Hilfe von Anzeigeinstrumenten überwacht, die an der Steuereinheit vorgesehen sind. Hierzu sind an der Saugglocke (1) ein Temperaturfühler (22) und ein Unterdruckfühler (23) vorgesehen. Die Temperierung der im Kaltwasserbehälter (19) und im Warmwasserbehälter (20) eingefüllten Medien (46, 45) erfolgt über Temperaturfühler (24, 25), die mit einer nicht dargestellten Regeleinheit verbunden sind. Wird die Steuereinheit auf automatischen Betrieb umgeschaltet, so werden die Behandlungsschritte von einer Ablaufsteuerung gesteuert. Die Ablaufsteuerung kann frei programmierbar ausgebildet sein. In einer ersten Betriebsphase werden die Ventile (13, 16) so angesteuert, daß ein Absaugen der Luft aus der Saugglocke (1) und das Ansaugen des ersten Behandlungsmediums erfolgt. In einer zweiten Betriebsphase steuert die Steuereinheit das Belüftungsventil (5) über die Induktionsspule (30) an, wodurch ein Leersaugen der Saugglocke (1) bei teilweise geöffneten Belüftungsventil (5) erfolgt. In einer dritten Betriebsphase werden wiederum die Ventile (13,16) angesteuert, wodurch ein Ansaugen eines zweiten Behandlungsmediums bewirkt wird.

Der eingestellte Temperaturbereich der Behandlungsmedien (45, 46) liegt zwischen 0°C und 43°C, wodurch eine Verletzungsgefahr durch falsche Wassertemperatur ausgeschlossen ist.Um ein Risiko durch den elektrischen Betrieb der Pumpe (15) auszuschließen, wird mit einem Gleichstrom gearbeitet, der zwischen 12 Volt und 48 Volt liegt.

Zur Sterilisation der gesamten Vorrichtung ist ein Sterilisationskreislauf vorgesehen, bei dem ein Medium mit hoher Temperatur, dem gegebenenfalls ein Sterilisationszusatzmittel beigesetzt ist, im Kreislauf geführt wird. Die notwendigen Sterilisationsintervalle können durch im normalen Kreislauf angeordnete Mikrowechselfilter verlängert werden.

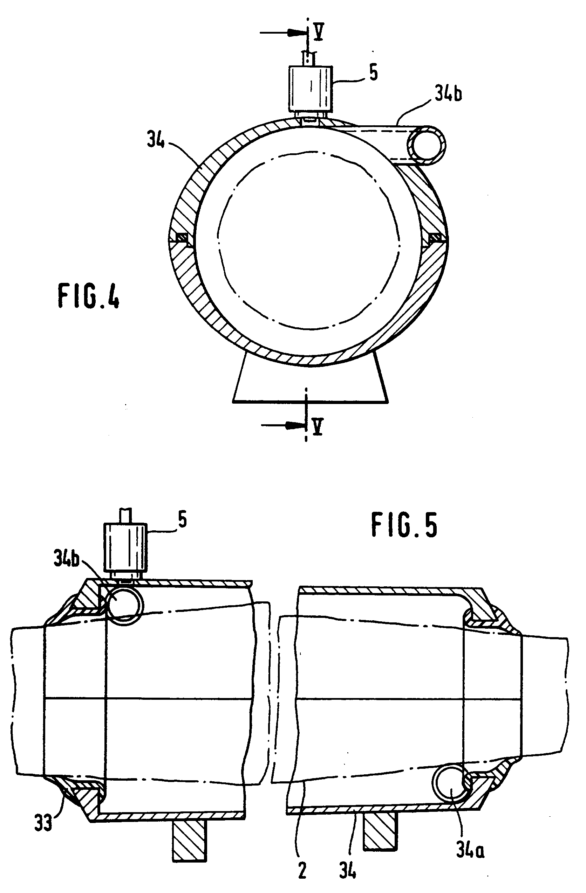

Eine andere Ausführungsform der Erfindung ist in den Fig. 4 und 5 dargestellt.Hierbei ist die Saugeinrichtung als Saugröhre (34) ausgebildet. Die Saugröhre (34) besitzt Zylinderform und ist an den Stirnseiten offen. Eine derartige Saugröhre (34) dient zur Behandlung eines Körpterteiles (2), z.B. eines Unterarmes.

An den Stirnseiten der Saugröhre (34) sind Dichtmanschetten (33) angeordnet, deren Dichtlippen an dem Körpterteil (2) anliegen. Die Saugröhre (34) besitzt einen Zulaufanschluß (34a), der in einem am tiefsten liegenden Bereich der Saugröhre (34) angeordnet ist, und einen Sauganschluß (34b), der in einem am höchsten liegenden Bereich der Saugröhre (34) positioniert ist. Um ein leichtes Anbringen an dem zu behandelnden Körperteil (2) zu ermöglichen, ist die Saugröhre (34) zweiteilig ausgebildet.

Die Saugröhre (34) kann mit sämtlichen Vorrichtungsmerkmalen kombiniert werden, die in Fig. 1 in Kombination mit der Saugglocke (1) dargestellt sind.

Eine vereinfachte Vorrichtung, die in der Zeichnung nicht dargestellt ist, kann man dadurch erreichen, daß für die beiden Behälter lediglich ein einziger Saugschlauch vorgesehen ist. Nach dem Einfüllen der Behandlungsmedien in die Behälter und der Kontrolle der erwünschten Temperatur durch ein eingebautes Thermometer wird der Saugschlauch in den ersten Behälter eingeführt. Der Saugschlauch ist am Zulaufanschluß einer Saugglocke angeordnet, die ohne Hebel, Schalter, Belüftungsventil und Meßfühler ausgebildet ist. Der einzige Ablaufschlauch ist am Sauganschluß der Saugglocke angeordnet. Über den Ablaufschlauch wird mittels einer Pumpe das Behandlungsmedium aus dem Behälter angesaugt. Die Betriebsphase mit einem anderen Behandlungsmedium wird durch manuelles Herausheben des einzigen Saugschlauches aus dem einen Behälter und das verzögerte Umlegen des einzigen Ablaufschlauches und danach des Saugschlauches in den anderen Behälter erreicht. Dies geschieht bei laufender Pumpe. Während dieses Vorganges muß die vereinfachte Saugglocke gegen die Oberfläche des Körpterteils gedrückt werden, um den nicht vorhandenen Unterdruck zu ersetzen. Eine derartige Vorrichtung ist sehr preiswert herzustellen.

## Patentansprüche

1. Vorrichtung zum Behandeln von Körperteilen eines Menschen, mit einer Saugeinrichtung (1,34), die an dem zu behandelnden Körperteil (2) ansetzbar ist, mit einem an der Saugeinrichtung vorgesehenen Sauganschluß (1b) und einem Zulaufanschluß (1a) für das Behandlungsmedium, mit einer Pumpe (15), die mit dem Sauganschluß (1b) verbunden ist,mit mindestens einem Behälter für das Behandlungsmedium und mit einer Heizvorrichtung (21) zum Erwärmen des Behandlungsmediums, gekennzeichnet durch einen weiteren Behälter (19) mit einem weiteren Behandlungsmedium (46), eine Kühlvorrichtung (17) zum Abkühlen des weiteren Behandlungsmediums (46), Schlauchverbindungen (3,4,26,27,28,29) zwischen der Saugeinrichtung (1,34), der Pumpe (15) und den Behältern (19,20) sowie Ventile (13,16), welche die für jede Betriebsphase erforderlichen Fließverbindungen herstellen, und ein an der Saugeinrichtung (1, 34) angeordnetes Belüftungsventil (5).

2. Vorrichtung nach Anspruch 1, dadurch gekennzeichnet, daß der Zulaufanschluß (1a, 34a) in einem am tiefsten liegenden Bereich der Saugeinrichtung (1, 34) und der Sauganschluß (1b, 34b) in einem am höchsten liegenden Bereich der Saugeinrichtung (1, 34) angeordnet sind.

3. Vorrichtung nach Anspruch 2, dadurch gekennzeichnet, daß das Belüftungsventil (5) im Bereich des Sauganschlusses (1b, 34b) angeordnet ist.

4. Vorrichtung nach einem der Ansprüche 1 bis 3, dadurch gekennzeichnet, daß das Belüftungsventil (5) zum Öffnen und Schließen von zwei Belüftungsbohrungen (7, 8) mit verschiedenem Querschnitt ausgebildet ist.

5. Vorrichtung nach Anspruch 4, dadurch gekennzeichnet, daß das Belüftungsventil (5) eine zentral angeordnete Ventilnadel (6) besitzt, die an einer in einen Schließstempel (31) vorgesehenen kleinen Bohrung (7) lösbar anliegt und im wesentlichen durch ihre Schwerkraft in Schließstellung gebracht ist.

6. Vorrichtung nach Anspruch 5, dadurch gekennzeichnet, daß der Schließstempel (31) des Belüftungsventils (5) an einer großen Bohrung (8), die an der Saugeinrichtung (1, 34) angebracht ist, und in Schließstellung von einem Federelement (9) gehalten ist, wobei die Betätigung des Schließstempels (31) mittels der Ventilnadel (6) und eines daran angeordneten Stiftes (32) erfolgt.

7. Vorrichtung nach den Ansprüchen 4 bis 6, dadurch gekennzeichnet, daß das Belüftungsventil (5) ansteuerbar und/oder manuell betätigbar ausgebildet ist.

8. Vorrichtung nach Anspruch 7, dadurch gekennzeichnet, daß im Belüftungsventil (5) eine mit der Ventilnadel (6) zusammenwirkende Induktionsspule (30) angeordnet ist.

9. Vorrichtung nach einem der Ansprüche 1 bis 8, dadurch gekennzeichnet, daß die Ventile (13, 16) ansteuerbar ausgebildet sind.

10. Vorrichtung nach den Ansprüchen 7 bis 9, dadurch gekennzeichnet, daß eine Steuereinheit vorgesehen ist, mit der die Ventile (13, 16), das Belüftungsventil (5) und die Pumpe (15) steuerbar sind.

11. Vorrichtung nach Anspruch 10, dadurch gekennzeichnet, daß die Steuereinheit manuell betätigbar ist.

12. Vorrichtung nach einem der Ansprüche 10 und 11, dadurch gekennzeichnet, daß die Steuereinheit mit einer automatischen Ablaufsteuerung und/oder Programmsteuerung versehen ist.

13. Vorrichtung nach den Ansprüchen 10 bis 12, dadurch gekennzeichnet, daß die Steuereinheit vom automatischen Betrieb auf manuellen Betrieb umschaltbar ist.

14. Vorrichtung nach den Ansprüchen 12 und 13, dadurch gekennzeichnet, daß die Steuereinheit eine Ablaufsteuerung besitzt, die in einer ersten Betriebsphase ein Absaugen der Luft aus der Saugeinrichtung (1, 34) und das Ansaugen des ersten Behandlungsmediums (45), in einer zweiten Betriebsphase ein Leersaugen der Saugeinrichtung (1, 34) bei teilweise geöffneten Belüftungsventil (5) und in einer dritten Betriebsphase ein Ansaugen eines zweiten Behandlungsmediums (46) bewirkt.

15. Vorrichtung nach einem der Ansprüche 10 bis 12, dadurch gekennzeichnet, daß die Ablaufsteuerung am Behandlungsende das Belüftungsventil (5) zunächst nur teilweise und dann zeitverzögert nach dem Absaugen des Behandlungsmediums (45, 46) vollständig öffnet.

16. Vorrichtung nach einem der Ansprüche 1 bis 15, dadurch gekennzeichnet, daß an der Saugeinrichtung (1, 34) ein Hebel (10) angelenkt ist, mit dem das Belüftungsventil (5) betätigbar ist.

17. Vorrichtung nach Anspruch 16, dadurch gekennzeichnet, daß die Ansteuerung der Ventile (13, 16) und der Pumpe (15) über Schalter (11, 35) erfolgt, die am Hebel (10) angeordnet sind oder mit Hilfe des Hebels (10) betätigbar sind.

18. Vorrichtung nach einem der Ansprüche 1 bis 17, dadurch gekennzeichnet, daß ein Mehrwegeventil (13) mit einer Vorlaufleitung (3), Steigleitungen (26,27) für die beiden Behandlungsmedien (45, 46) und mit einer in eine Rücklaufleitung (4) führende Verbindungsleitung (14) verbunden ist.

19. Vorrichtung nach einem der Ansprüche 1 bis 18, dadurch gekennzeichnet, daß ein Zweiwegeventil (16) an einer Druckseite (39) der Pumpe (15) angeordnet ist.

20. Vorrichtung nach einem der Ansprüche 1 bis 19, dadurch gekennzeichnet, daß an den Behälter (19, 20) eine Temperaturregelung für die Behandlungsmedien (45, 46) vorgesehen ist.

21. Vorrichtung nach einem der Ansprüche 1 bis 20, dadurch gekennzeichnet, daß die Saugeinrichtung als teiloffene Saugglocke (1) ausgebildet ist.

22. Vorrichtung nach einem der Ansprüche 1 bis 21, dadurch gekennzeichnet, daß die Saugeinrichtung als Saugröhre (34) ausgebildet ist.

23. Vorrichtung nach Anspruch 22, dadurch gekennzeichnet, daß an stirnseitigen Öffnungen der Saugröhre (34) eine Dichtmanschette (33) angeordnet ist.

## Claims

1. An apparatus for the treatment of parts of the human body, with a suction arrangement (1, 34) which can be applied to the part (2) of the body which is to be treated, with a suction connection (1b) provided on the suction means and with a feed connection (1a) for the treatment medium, with a pump (15) which is connected to the suction connection (1b), with at least one container for the treatment medium and with a heating device (21) for heating the treatment medium, characterised by a further container (19) with a further treatment medium (46), a cooling device (17) for cooling the further treatment medium (46), hose connections (3, 4, 26, 27, 28, 29) between the suction means (1, 34) of the pump (15) and the containers (19, 20) and valves (13, 16), which establish the flow connections required for each operating phase and a ventilating valve (5) disposed on the suction means (1, 34).

2. An apparatus according to Claim 1, characterised in that the feed connection (1a, 34a) is disposed in the very lowest part of the suction means (1, 34) while the suction connection (1b, 34b) is disposed in the highest part of the suction means (1, 34).

3. An apparatus according to Claim 2, characterised in that the ventilating valve (5) is disposed in the region of the suction connection (1b, 34b).

4. An apparatus according to one of Claims 1 to 3, characterised in that the ventilating valve (5) is constructed for opening and closing to ventilating ports (7, 8) having different cross-sections.

5. An apparatus according to Claim 4, characterised in that the ventilating valve (5) has a centrally disposed valve needle (6) which rests removably in a small bore (7) provided in a closing ram (31) and is substantially brought to its closed position by force of gravity.

6. An apparatus according to Claim 5, characterised in that the closing ram (31) of the ventilating valve (5) is held in a large bore (8) provided in the suction means (1, 34) and in the closed position by a spring element (9), the closing ram (31) being actuated by the valve needle (6) and by a rod (32) disposed thereon.

7. An apparatus according to Claims 4 to 6, characterised in that the ventilating valve (5) can be controlled and/or is constructed to be actuated manually.

8. An apparatus according to Claim 7, characterised in that an induction coil (30) is disposed in the ventilating valve (5) and cooperates with the valve needle (6).

9. An apparatus according to one of Claims 1 to 8, characterised in that the valves (13, 16) are constructed to be controllable.

10. An apparatus according to Claims 7 to 9, characterised in that a control unit is provided by which it is possible to control the valves (13, 16), the ventilating valve (5) and the pump (15).

11. An apparatus according to Claim 10, characterised in that the control unit can be manually actuated.

12. An apparatus according to one of Claims 10 and 11, characterised in that the control unit is provided with an automatic cycle control and/or programme control.

13. An apparatus according to Claims 10 to 12, characterised in that the control unit can be switched over from automatic operation to manual operation.

14. An apparatus according to Claims 12 and 13, characterised in that the control unit has a cycle control which in a first phase of operation causes air to be sucked in from the suction means (1, 34) and a drawing in of the first treatment medium (45) while in a second operating phase it produces an emptying of the suction means (1, 34) by suction with a partially opened ventilating valve (5) and in a third phase of operation it causes a drawing in of a second treatment medium (46) by suction.

15. An apparatus according to one of Claims 10 to 12, characterised in that when treatment finishes, the cycle control opens the ventilating valve (5) firstly only partially and then, after a delay, completely after extraction of the treatment medium (45, 46) by suction.

16. An apparatus according to one of Claims 1 to 15, characterised in that a lever (10) by which the ventilating valve (5) can be actuated is articulated on the suction means (1, 34).

17. An apparatus according to Claim 16, characterised in that the valves (13, 126) and the pump (15) are operated via switches (11, 35) which are disposed on the lever (10) or which can be actuated by means of the lever (10).

18. An apparatus according to one of Claims 1 to 17, characterised in that a multi-way valve (13) is connected to a primary flow pipe (3), riser pipes (26, 27) for the two treatment media (45, 46) and to a connecting pipe (14) leading to a return pipe (4).

19. An apparatus according to one of Claims 1 to 18, characterised in that a two-way valve (16) is disposed on a pressure side (39) of the pump (15).

20. An apparatus according to one of Claims 1 to 19, characterised in that there is on the containers (19, 20) an arrangement for controlling the temperature of the treatment media (45, 46).

21. An apparatus according to one of Claims 1 to 20, characterised in that the suction means is constructed as a partially-open suction bell (1).

22. An apparatus according to one of Claims 1 to 21, characterised in that the suction means is constructed as a suction tube (34).

23. An apparatus according to Claim 22, characterised in that a sealing sleeve (33) is disposed on end apertures of the suction tube (34).

## Revendications

1. Dispositif pour le traitement des parties du corps humain incluant un dispositif d'aspiration (1, 34) qui peut être appliqué sur la partie (2) à traiter du corps, le dispositif d'aspiration comprenant une entrée d'aspiration (1b) et une sortie d'aspiration (1a) pour le medium à traiter avec une pompe (15) qui est reliée à l'entrée d'aspiration (1b), au moins un récipient pour le medium à traiter et un dispositif de chauffage (21) pour réchauffer le medium caractérisé en ce qu'il comprend en outre un autre récipient (19) avec un autre dispositif de traitement (46), un dispositif de refroidissement (17) pour refroidir le second medium à traiter (46), des canalisations de liaison (3, 4, 26, 27, 28, 29) entre les dispositifs d'aspiration (1, 34), la pompe (15) et les récipients (19, 20) et des soupapes (13, 16) qui, pour chaque phase de travail, établissent des liaisons de flux et, sur l'installation d'aspiration (1, 34) est installée une soupape de ventilation (5).

2. Dispositif selon la revendication 1, caractérisé en ce que les sorties (1a, 34a) sont disposées dans la région la plus basse du dispositif (1, 34) et les entrées (1b, 34b) dans la région la plus haute du dispositif (1, 34).

3. Dispositif selon la revendication 2, caractérisé en ce que la soupape de ventilation (5) est disposée au voisinage de l'entrée (1b, 34b).

4. Dispositif selon l'une quelconque des revendications 1 à 3, caractérisé en ce que la soupape de ventilation (5) est conçue pour ouvrir et fermer deux trous d'aération (7, 8) présentant des sections différentes.

5. Dispositif selon la revendication 4, caractérisé en ce que la soupape de ventilation (5) présente une aiguille centrale (6) qui repose sur un siège de fermeture (31) par un petit trou (7) et qui est rappelé en position de fermeture par pesanteur.

6. Dispositif selon la revendication 5, caractérisé en ce que le siège (31) de la soupape de ventilation (5) repose sur un trou (8) plus grand qui est prévu dans le dispositif d'aspiration (1, 34) et, en position de fermeture, est maintenu par un ressort de rappel (9) de sorte que l'action du siège de fermeture (31) s'effectue au moyen de l'aiguille de soupape (6) et d'une collerette (32).

7. Dispositif selon l'une quelconque des revendications 1 à 6, caractérisé en ce que la soupape de ventilation (5) est manoeuvrable automatiquement ou manuellement.

8. Dispositif selon la revendication 7, caractérisé en ce que la soupape de ventilation (5) est montée avec une aiguille de soupape (6) à l'intérieur d'une bobine d'induction (30).

9. Dispositif selon l'une quelconque des revendications 1 à 8, caractérisé en ce que les soupapes (13, 16) sont automatiques.

10. Dispositif selon l'une quelconque des revendications 7 à 9, caractérisé en ce que une unité de commande est prévue pour la commande des soupapes (13, 16), la soupape d'aération et la pompe (15).

11. Dispositif selon la revendication 10, caractérisé en ce que l'unité de commande est manoeuvrable manuellement.

12. Dispositif selon les revendications 10 et 11, caractérisé en ce que l'unité de commande contient une commande automatique de départ et/ou un programme.

13. Dispositif selon l'une quelconque des revendications 10 à 12, caractérisé en ce que l'unité de commande peut être commutée du fonctionnement automatique au fonctionnement manuel.

14. Dispositif selon les revendications 12 et 13, caractérisé en ce que l'unité de commande consiste en une commande de démarrage qui dans une première phase consiste dans une aspiration de l'air hors du dispositif d'aspiration (1, 34) et le remplissage des premiers médiums à traiter (45), et dans une seconde phase, dans un vidage de dispositif d'aspiration (1, 34) par la partie ouverte de la soupape de ventilation (5), et dans une troisième phase, en un remplissage par un second médium à traiter (46).

15. Dispositif selon l'une quelconque des revendications 10 à 12, caractérisé en ce que la commande de démarrage à la fin du traitement la soupape de ventilation (5) reste complètement ouverte après le remplissage des médiums à traiter.

16. Dispositif selon l'une quelconque des revendications 1 à 15, caractérisé en ce que sur le dispositif d'aspiration (1, 34) est articulé un levier (10) qui est manoeuvrable avec la soupape de ventilation.

17. Dispositif selon la revendication 16, caractérisé en ce que la commande des soupapes (13, 16) et de la pompe (15) suit des commutateurs (11, 35) qui sont montes sur le levier (10) ou qui est actionnable à l'aide du levier (10).

18. Dispositif selon l'une quelconque des revendications 1 à 17, caractérisé en ce que une vanne multi-voies (13) avec un conduit d'entrée (3) des conduits montants (26, 27) pour les deux médiums à traiter et un conduit de sortie (4), est reliée au conduit (14).

19. Dispositif selon l'une quelconque des revendications 1 à 18, caractérisé en ce que une vanne à deux voies (16) est montée à la sortie (39) de la pompe (15).

20. Dispositif selon l'une quelconque des revendications 1 à 19, caractérisé en ce que une régulation de température pour les médiums à traiter (45, 46) est prévue dans les récipients (19, 20).

21. Dispositif selon l'une quelconque des revendications 1 à 20, caractérisé en ce que le dispositif d'aspiration constitue une partie ouverte d'une cloche d'aspiration (1).

22. Dispositif selon l'une quelconque des revendications 1 à 21, caractérisé en ce que le dispositif d'aspiration constitue un tube d'aspiration (34).

23. Dispositif selon la revendication 22, caractérisé en ce qu'une collerette d'étanchéité (33) est montée sur les ouvertures latérales du tube d'aspiration (34).
